# EUROPEAN PATENT APPLICATION

(11) **EP 3 040 412 A1**
(43) Date of publication of application: **06.07.2016**
(21) Application number: 14837645.2
(22) Date of filing: 21.08.2014
(51) Int. Cl.: C12N 1/20, A61K 35/74, A23L 33/10, C12R 1/225

(54) **PROBIOTIC STRAIN OF LACTOBACILLUS REUTERI EXHIBITING ANTI-HELICOBACTER ACTIVITY, A PROBIOTIC OR BIOTHERAPEUTIC PRODUCT COMPRISING SAID STRAIN, AND THE USE THEREOF**

(30) Priority: 23.08.2013 ES 201331271
(71) Applicant: Consejo Superior De Investigaciones Científicas (CSIC), 28006 Madrid (ES)
(72) Inventor: MAYO PÉREZ, Baltasar, E-33300 Villaviciosa (Asturias) (ES); DELGADO PALACIO, Susana, E-33300 Villaviciosa (Asturias) (ES)
(74) Representative: Myers, Jonathan David
(86) International application number: PCT/ES2014/070666
(87) International publication number: WO 2015/025072

(57) **Abstract**

The invention relates to a new strain of *Lactobacillus reuteri* isolated from the human stomach, exhibiting antioxidant and antimicrobial activity, in particular an *anti -Helicobacter* effect, considerable resistance to the conditions of the gastric ecosystem, and the capacity to adhere to the epithelium. The invention also relates to a probiotic or biotherapeutic product which contains the strain of *L. reuteri* and is suitable for use in the treatment or prophylaxis of gastric diseases caused by the *Helicobacter pylori* bacteria, in order to prevent the secondary effects associated with conventional antibiotic treatments.

## Description

### Field of the Art

The present invention is encompassed in the field of biotechnology and is applicable in the sectors of food industry and biomedicine, and more specifically in the field of using probiotic microorganisms with prophylactic or palliative nature with respect to gastric pathologies caused by bacterial dysbioses and/or *Helicobacter pylori* infections.

### State of the Art

*H. pylori* is a Gram-negative microorganism colonizing the human duodenal and gastric epithelium, occasionally invading epithelial cells. Chronic *H. pylori* infections can give rise to gastritis, peptic and duodenal ulcers, stomach cancer and MALT (mucosa-associated lymphoid tissue) lymphoma (Peek and Blaser, 2002; Nat. Rev. Cancer 2:28-37). *H. pylori* infection induces oxidative stress by causing an increase in reactive oxygen species (or ROS) such as peroxides and free radicals damaging proteins, lipids and DNA, which could be linked to the carcinogenic capacity of the microorganism. *H. pylori* also causes a decrease in cell anti-oxidative defense systems responsible for detoxifying the ROS and/or repairing the resulting damage (Hütt et al., 2009; Lett. Appl. Microbiol. 48:797-800).

Today, there are different types of strategies to deal with the treatment of *H. pylori* infections. The eradication rate of this pathogen depends on the combination of antibiotics and antacids with efficiency varying between 80 and 90%. The conventional first-line therapy is one week of broad spectrum triple therapy consisting of antibiotherapy with amoxicillin and clarithromycin, and a proton pump inhibitor as recommended by the European and American guidelines (Malfertheiner et al., 2007; Gut 56:772-781), with the corresponding side effects of using such antibiotics.

Variations of the triple therapy have been developed over the years. It has particularly been found that more and more infected individuals have antibiotic resistant bacteria. This results in the failure of the initial treatment and requires additional rounds of therapies with antibiotics or alternative strategies such as a quadruple therapy. Among the collateral adverse effects associated with antibiotic treatment is a considerable reduction of the natural endogenous microbiota. For this reason, new or alternative treatments which increase tolerance and efficacy, as well as the pathogen eradication rates are being investigated. The 2007 report "Maastricht III Consensus Report on *H. pylori"* already points to the use of probiotics as a possible tool for managing these infections (Malfertheiner et al., 2007; Gut 56:772-781), for alleviating the effects derived from antibiotherapy and re-establishing natural microbiota. In fact, many *in vitro* studies have demonstrated the capacity of several strains from the genus *Lactobacillus* in reducing growth and inhibiting adhesion to the mucosa of *H. pylori.* Studies in model animals and in humans have also clearly shown the ability of various strains of lactobacilli in preventing and/or reducing *Helicobacter* infection and colonization (Sykora et al., 2005; J. Clin. Gastroenterol. 39:692-698).

*Lactobacillus reuteri* is a heterofermentative microorganism residing as a symbiont in the human gastrointestinal tract and in different mammals. In fact, it is considered one of the few truly autochthonous or endogenous lactobacillus species in humans. The existence of strains of this species that are highly specialized or adapted to the niche and host has been shown recently (Oh et al., 2010; ISME J. 4(3): 377-387).

Various studies have been carried out for the purpose of evaluating the effect of using the strains *of L. reuteri* in patients with *H. pylori.* These studies have proven that administration of *L. reuteri* as a therapeutic adjuvant induces pathogen eradication and improves the dyspeptic symptomatology (Zou et al., 2009; Helicobacter 14:97-107). Specifically, the strain *L. reuteri* ATCC 55730 (also known as SD2112) is widely studied and commonly used as food supplement or additive to improve gastrointestinal health.

Documents protecting strains of *L. reuteri* are also recorded in the patent literature. Patent document US2004/0067573 A1, for example, relates to the capacity of the strain *L. reuteri* MM2-3, ATCC PTA-4659, to reduce the inflammation caused by *H. pylori,* as well as to food and pharmaceutical compositions containing it and to the use thereof for the treatment or prophylaxis of *H. pylori.*

In turn, patent document EP20100460046 relates to the strain *L. reuteri* DAN080 and to the use thereof for the prophylaxis and treatment of infections caused by pathogens of the gastrointestinal tract such as *H. pylori* in mammals and poultry.

However, the search and production of new strains isolated from the host itself and in particular from the gastric environment, such as that proposed in this invention, allows producing new microorganisms with differentiated characteristics and with capacities greater than those of other microorganisms present in the state of the art, making them more appealing for combating stomach pathogens such as *H. pylori.*

### Description of the Invention

### Brief Description of the Invention

The technical problem to be solved is the search for a solution to maintain bacterial homeostasis in the gastric ecosystem for the purpose of maintaining and prolonging the state of health of this organ by means of using probiotic strains. Alternatively, the strains could be used for improving the prophylaxis and treatment of gastric pathologies caused by bacterial dysbioses and/or *H. pylori* infections.

The first object of the invention relates to the probiotic strain of *Lactobacillus reuteri* LR32 deposited on July 4, 2013 in the Spanish Type Culture Collection (CECT) with accession number CECT 8395, following the Budapest Treaty on the International Recognition of the Deposit of Microorganisms for the Purposes of Patent Procedure, isolated from the human stomach, with the capacity to colonize and survive in the conditions of the human gastric ecosystem, which is characterized by having:
a) a percentage of adherence to the gastric epithelium greater than 5%,
b) a survival rate at pH 2, in conditions of anaerobiosis, greater than 80%, and
c) resistance to the presence of up to 4% of bile after an incubation period of 48 h at a temperature of 37°C.

In one aspect of the invention, the probiotic strain of *Lactobacillus reuteri* LR32 has a total *in vitro* anti-oxidative activity greater than 20%.

In another aspect of the invention, the probiotic strain of *Lactobacillus reuteri* LR32 has an antimicrobial capacity which is derived from:
ii) its capacity to produce reuterin, and
iii) its capacity to produce hydrogen peroxide in conditions of aerobiosis and anaerobiosis.

In another aspect of the invention, the probiotic strain of *Lactobacillus reuteri* LR32 has the capacity to inhibit *H. pylori.*

The second object of the invention relates to the use of the probiotic strain of *Lactobacillus reuteri* LR32 for preventing and/or alleviating gastric pathologies derived from microbial dysbioses, preferably those caused by the bacterium *H*. *pylori* as well as the side effects derived therefrom.

The third object of the invention relates to the use of the probiotic strain of *Lactobacillus reuteri* LR32 for preparing a food composition, a food supplement or a biotherapeutic composition useful for preventing and/or alleviating gastric pathologies derived from microbial dysbioses, preferably those caused by the bacterium *H. pylori* as well as the side effects derived therefrom.

Finally, the fourth object of the invention relates to a food composition, a food supplement or a biotherapeutic composition comprising the probiotic strain.

### Detailed Description of the Invention

The present invention is based on the observation that a microorganism belonging to a new strain of the species *Lactobacillus reuteri,* which has been isolated from the human stomach of a healthy subject (see Example 1), has the capacity to survive in and colonize the gastric ecosystem (see Examples 2 and 3), antimicrobial capacity, particularly anti-*H*. *pylori* effect (see Examples 4, 5 and 6) and anti-oxidative activity (see Example 7). These capacities allow the use thereof as a microbial homeostatic agent and in the prophylaxis or treatment of gastric pathologies caused by microbial dysbioses and/or associated with *H. pylori* overgrowth.

The main technical advantages of the probiotic strain of *L*. *reuteri* being protected are listed below:
a) capacity to colonize the gastric ecosystem, derived from its origin, and reflected through the percentages of adhesion to a human gastric epithelial line, which are greater than those of, for example, the commercial probiotic strain *Lactobacillus rhamnosus* GG widely used as probiotic,
b) resistance to conditions with an extremely acidic pH and to bile salts secreted by the duodenum, typical of the lower part of the stomach colonized by the pathogen *H. pylori,*
c) anti-oxidative activity, preventing lipid peroxidation in a percentage greater than that of other gastric strains of lactobacilli and within the range of acceptable values for strains exhibiting proven anti-oxidative activity,
d) antimicrobial capacity based on the combination of:
   - its potential to produce reuterin (potent antimicrobial agent with the capacity to inhibit the growth of a wide range of microorganisms, including Gram-positive and Gram-negative bacteria);
   - its capacity to produce hydrogen peroxide (H₂O₂) which is an antimicrobial agent that aids in the colonization of a specific ecosystem, favoring the competence of the producer microorganism for space and nutrients;
   - its antagonistic capacity against *H. pylori,* with reduction rates of this pathogen significantly greater than those of other strains of lactobacilli of the gastric environment, and
e) capacity to grow in and acidify UHT milk, which enables it to develop in fermented dairy products derived therefrom.

The first object of the invention relates to a probiotic strain isolated from the human stomach, with the capacity to colonize and survive in the gastric ecosystem, hereinafter the probiotic strain of the invention, which is characterized by having:
a) a percentage of adherence to the gastric epithelium greater than 5%,
b) a survival rate at pH 2, in conditions of anaerobiosis, greater than 80%, and
c) resistance to the presence of up to 4% of bile after an incubation period of 48 h at a temperature of 37°C.

In the present invention, "probiotic strain" is understood as a microorganism of a specific genus and species, preferably *Lactobacillus reuteri,* which when taken in suitable amounts can have a beneficial effect on the host health.

A particular aspect of the invention relates to the probiotic strain of the invention with a survival rate at pH 2.5, in conditions of anaerobiosis, greater than 95%.

The probiotic strain of the invention has an anti-oxidative activity preventing lipid peroxidation and thereby reactive oxygen species accumulation.

In another particular aspect of the invention, the probiotic strain of the invention has a total *in vitro* anti-oxidative capacity greater than 20%.

"Total anti-oxidative activity" is understood as an overall estimation (expressed in the form of percentage) of the capacity of a system (food, microorganism, etc.) to prevent oxidation and reduce free radical formation.

In another aspect of the invention, the probiotic strain of the invention has an antimicrobial activity, characterized by:
i) its capacity to produce reuterin, and
ii) its capacity to produce hydrogen peroxide in conditions of aerobiosis and anaerobiosis.

The probiotic strain of the invention contains the key gene related to reuterin production. Reuterin is a potent antimicrobial agent produced during anaerobic metabolism of glycerol, exhibiting inhibitory activity even against Gram-negative microorganisms such as *H. pylori.*

In the present invention, "reuterin" is understood as an antimicrobial compound produced by *Lactobacillus reuteri* while metabolizing glycerol in conditions of anaerobiosis.

*"Helicobacter pylori"* is understood as a particular type of Gram-negative microorganism colonizing and infecting the human gastric and duodenal epithelium, being able to cause gastritis, ulcers and cancer.

"Hydrogen peroxide (H₂O₂)" is understood as a potent antimicrobial agent produced by various lactobacillus species isolated from the human stomach.

The probiotic strain of the invention has a significant *anti-Helicobacter* activity in inhibition assays in liquid medium.

In a particular embodiment of the invention, the probiotic strain of the invention has a capacity to inhibit *H. pylori* in non-neutralized supernatant of 75%.

In another particular embodiment of the invention, the probiotic strain of the invention has a capacity to inhibit *H*. *pylori* in neutralized supernatant of 55%.

The capacity to inhibit the pathogen in non-neutralized supernatant is fundamentally related to the production of organic acids, whereas the capacity to inhibit same in the neutralized supernatant of the probiotic strain of the invention indicates that the inhibitory effects is based on the production of another type of antimicrobial compound/compounds (bacteriocins, reuterin, H₂O₂, etc.).

In a preferred embodiment of the invention, the probiotic strain of the invention is a strain of lactic acid type bacteria, preferably belonging to the genus *Lactobacillus,* and more preferably the species *Lactobacillus reuteri.*

In another particular embodiment of the invention, the probiotic strain of the invention is, without any limitation, a cell of the probiotic strain, a cell fragment of the probiotic strain, an extract of the bacterial strain or a purified component of the probiotic strain, present in the form of concentrate or lyophilisate.

In a more preferred embodiment of the first aspect of the invention, the probiotic strain of the invention is the strain *Lactobacillus reuteri* LR32 deposited on July 4, 2013 in the Spanish Type Culture Collection (CECT) with accession number CECT 8395, following the Budapest Treaty on the International Recognition of the Deposit of Microorganisms for the Purposes of Patent Procedure.

*Lactobacillus reuteri* LR32 comprises a partial sequence of the gene encoding the 16S rRNA, amplified with the oligos 27F and 1492R and sequenced with the primer corresponding with SEQ ID NO: 1.

The isolation of other similar strains of *Lactobacillus,* based on the information described in the present invention can be used and carried out by a person skilled in the art, thereby expanding the strains with similar characteristics that can be used commercially and form part of the present invention.

The second object of the invention relates to the use of the probiotic strain of the invention for preventing and/or alleviating gastric pathologies derived from different microbial dysbioses, and preferably those caused by the bacterium *H*. *pylori.*

"Microbial dysbiosis" is understood as the deregulation of microbial populations co-habiting in a specific niche or ecosystem, such that the respective proportions thereof are altered modifying the normal microbial balance necessary for maintaining health and wellbeing.

The probiotic strain of the invention can be used for preparing food products and/or biotherapeutic products.

The third object of the invention relates to the use of the probiotic strain of the invention for preparing a food composition, a food supplement or a biotherapeutic composition useful for preventing and/or alleviating gastric pathologies derived from microbial dysbioses, preferably those caused by the bacterium *H. pylori* as well as the side effects derived therefrom.

A fourth object of the invention relates to a probiotic food composition, a food supplement or another type of biotherapeutic composition which comprises the probiotic strain of the invention and is useful for preventing and/or alleviating gastric pathologies derived from different microbial dysbioses, and preferably those caused by the bacterium *H. pylori.*

"Probiotic food composition" is understood as any combination of ingredients making up a solid or liquid food product, which in addition to its nutritional characteristics, comprises specific functions aiding to improve health and reduce the risk of contracting illnesses.

"Food supplement" is understood as an additional nutritional supplement intended for complementing the diet.

"Biotherapeutic composition" is understood as the product consisting of a microbial preparation with a beneficial effect on health or therapeutic effect.

The resistance of the probiotic strain of the invention to conditions of extreme acidity makes it particularly suitable for being used in conditions of acidic pH and particularly in fermented products.

The probiotic strain of the invention is developed in milk reaching levels of 10⁸ colony forming units (cfu)/ml, and acidifies the product in a manner comparable to that carried out by the strains of yogurt.

One embodiment of the fourth aspect of the invention relates to a food composition of the invention which is a fermented product, and preferably a dairy product fermented exclusively by the probiotic strain of the invention.

Another embodiment of the fourth aspect of the invention relates to the food composition of the invention, which is a fermented product using other microorganisms for fermentation, and in which the probiotic strain of the invention is included as adjunct culture.

Another embodiment of the fourth aspect of the invention relates to the food supplement according to any one of the following presentations, in the form of capsules, in lyophilisate form, in liquid form, in the form of pills or in the form of gels.

Another embodiment of the fourth aspect of the invention relates to the biotherapeutic composition according to any one of the following presentations, in the form of capsules, in lyophilisate form, in liquid form, in the form of pills or in the form of gels.

Throughout the description and claims the word "comprises" and the variants thereof do not intend to exclude other technical features, additives or steps. For the person skilled in the art, other aspects, advantages and features of the invention will be inferred in part from the description and in part from putting the invention into practice. The following drawings and examples are provided by way of illustration and do not seek to limit the present invention.

### Brief Description of the Drawings

Figure 1 shows the recognition of the strain *Lactobacillus reuteri* LR32. The recognition was carried out by means of the restriction gene profile thereof with the enzyme *Sma*l*.*
Figure 2 shows adhesion to the gastric epithelial line AGS. The strains *L. reuteri* L32 and *L. reuteri* L34 isolated from the human stomach and the commercial probiotic strain *Lactobacillus rhamnosus* GG were used according to Example 2.
Figure 3 shows the survival at an acidic pH of different strains of lactobacilli. The strains *Lactobacillus reuteri* L32, *L. reuteri* L34 and the strain *L. rhamnosus* GG were used, after a 90-minute exposure in a solution with hydrochloric acid (HCl) at pH 2.5 and pH 2, according to Example 3.2.
Figure 4 shows the inhibition of *H. pylori* by supernatants. Different gastric strains of *Lactobacillus,* including *L. reuteri* LR32 were used according to Example 4.
Figure 5 shows the partial PCR amplification of the glycerol dehydratase gene essential for the production of reuterin in the strain *L. reuteri* LR32. Order of the tested strains: 1, *L. reuteri* 925 (positive control strain); 2, *L. reuteri* LR32; 3, *L. reuteri* LR34; 4, *Lactobacillus fermentum* LF72; 5, *Lactobacillus vaginalis* LV121; 6, *Lactobacillus gasseri* LG102; 7, *L. gasseri* LG52. M, molecular weight marker.
Figure 6 shows the production of hydrogen peroxide (H₂O₂). Different strains of *Lactobacillus* isolated from the human stomach including *L. reuteri* LR32 (in the center) were used according to Example 6.
Figure 7 shows the growth and acidification of UHT milk by the strain *L. reuteri* LR32.

### Embodiments of the Invention:

Several examples illustrating the isolation of the probiotic strain of the invention and its capacity to survive in and colonize the gastric ecosystem, as well as its antimicrobial capacity and anti-oxidative activity, which do not however limit the present invention, are described below.

### Example 1. Isolation of the strain of Lactobacillus reuteri LR32 from the human stomach

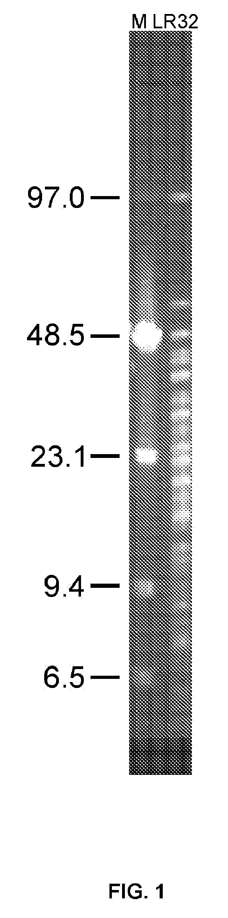

The strain of *L. reuteri* LR32 was isolated from a mucosal sample of the stomach of a healthy subject taken during a routine gastric endoscopy (Delgado et al., 2013; Microb. Ecol. 65:763-72). After biopsy, the mucosal sample was delivered to the laboratory in a sterile reducing solution made up of 0.9% NaCl, 0.1% peptone, 0.1% Tween 80® and 0.02% cysteine, being analyzed within two hours following extraction. The sample was washed in phosphate buffered saline (PBS) and homogenized and broken up in a maximum recovering diluent (Scharlab). This suspension (100 µl) was seeded in duplicate on the surface of MRSC ("de Man, Rogosa and Sharpe" medium with 0.25% cysteine) culture plates. The strain LR32 was recovered from these plates after 72 hours of incubation in an anaerobic chamber (Mac500, Down Whitley Scientific) with anaerobic atmosphere (10% H₂, 10% CO₂, 80% N₂). The strain was reseeded to depletion in the same medium for purification and re-inoculated in liquid medium for storage thereof at 80°C in the presence of 30% glycerol. For the molecular identification thereof, the gene encoding the 16S rRNA was amplified by PCR by means of using the universal primers 27F and 1492R, the amplicon being partially sequenced (SEQ ID NO: 1). Furthermore, the strain of *L. reuteri* was subjected to pulsed-field gel electrophoresis to establish its macrorestriction profile. To that end, the genomic DNA of the strain was isolated and digested in agarose blocks with 20U of the enzyme *Sma*l (Boehringer) at 30°C for 4 hours. The electrophoresis was carried out with pulses of 0.1-2 s 4 h, 2-5 s 12 h and 5-10 s 4 h. The strain LR32 is thereby differentiated from other strains of the same species by means of the restriction gene profile, as seen in Figure 1.

### A. Capacity of the strain L. reuteri LR32 to survive in conditions of the gastric ecosystem

### Example 2. Capacity of the strain of L. reuteri LR32 and of other strains used as reference to adhere to the gastric epithelium

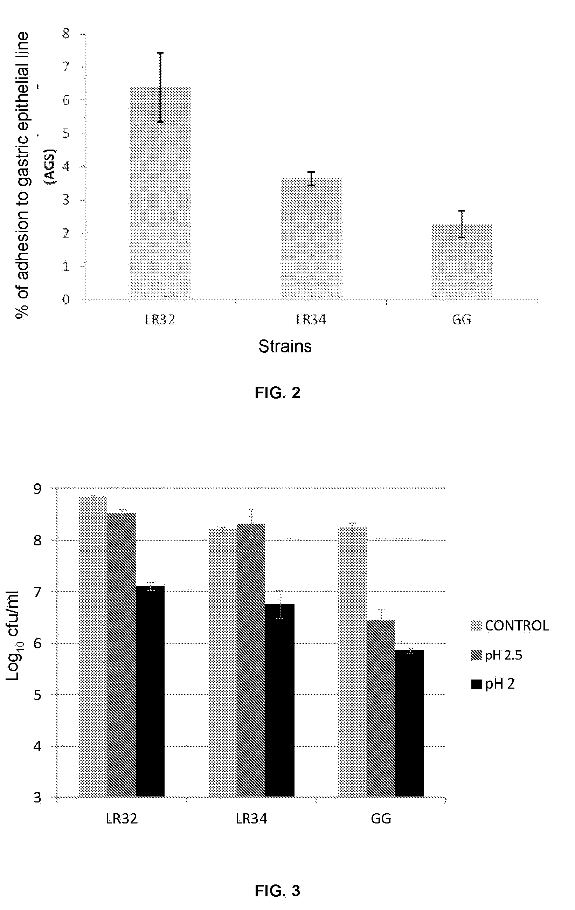

The experimental procedure consisted of co-incubating the epithelial line AGS in confluent phase with the strain *L*. *reuteri* LR32 obtained according to Example 1. Additionally, the epithelial line was co-incubated with another gastric strain of *L. reuteri* (LR34) and with the commercial probiotic strain *Lactobacillus rhamnosus* GG (considered an adherent strain standard). A concentration of 10⁵ cfu/ml of each bacterial strain and a bacteria:cells ratio of 1:1 were used. The adhesion assays were performed in wells in duplicate and the experiment was repeated three times. After one hour of joint incubation at 37°C, bacteria that did not adhere to the epithelial monolayer were eliminated with three successive washes with PBS. The adhered bacteria were detached by breaking the monolayer with a 0.25% trypsin-EDTA solution and counted by means of ten-fold serial dilutions and MRSC plate counts. The results were expressed as the percentage of bacteria adhered with respect to the concentration of bacteria added at the beginning (see Figure 2), the mean of the different assays and the standard deviation being calculated. The adherence capacity of the strain of *L*. *reuteri* LR32 was compared with the adherence of the other strain of *L. reuteri* (LR34) and with that of *L. rhamnosus* GG. As can be seen in Figure 3, the adherence of the strain *L. reuteri* LR32 was 3 times greater than that of the standard strain *L*. *rhamnosus* GG, and 1.75 times greater than that of the other strain of *L. reuteri.*

### Example 3. Resistance of the strain of L. reuteri LR32 isolated from the human stomach and of other strains used as reference to the conditions of the gastrointestinal tract

To see the resistance capacity of the strain *L. reuteri* LR32 isolated from the human stomach and of other strains that were used as reference, the strains were subjected to conditions of extreme acidity and to the presence of intestinal bile salts to simulate conditions similar to those of the upper part of the gastrointestinal tract.

### 3.1. Capacity to grow in acidic conditions

The strain *L. reuteri* LR32 of the invention and another gastric strain of *L. reuteri* (LR34) were subjected to a preliminary assay to determine their capacity to initiate growth at pH 2.5. To that end, based on the active 24-hour cultures of the strains "Microtiter" plates filled with 200 µl MRSC medium acidified with 12N hydrochloric acid (HCl) at pH 2.5 were inoculated by 2%. The plates were incubated in anaerobic chamber and after 48 hours, the presence of growth "button" was observed. Non-acidified MRSC was used as control (pH 5.7). Both strains of *L. reuteri* were capable of initiating growth in MRSC broth at pH 2.5.

### 3.2. Survival with respect to acidity after a 90-minute incubation

The strain of *L. reuteri* LR32 obtained according to Example 1, another strain of *L. reuteri* (LR34) and the commercial probiotic strain *Lactobacillus rhamnosus* GG were grown in MRSC broth, the concentration thereof being adjusted to 10⁸ cfu/ml in sterile saline solution. The tolerance to acid was tested after a 90-minute exposure of the strains to solutions acidified to pH 2.5 and pH 2.0. Additionally, a control consisting of a non-acidified saline solution was used. After 90 min of incubation at 37°C in the acidified physiological solution, the number of viable bacteria was estimated by means of MRSC plate counts. The results (see Figure 3) showed how the viability of the strain LR32, as well as the strain LR34, was greater at pH 2.5 than at pH 2.0. At pH 2.5 both strains showed a minimum reduction in the starting bacterial population. At pH 2.0 the decrease in the counts was more obvious, at about 1.5 log units but maintaining high viability levels (about 10⁷ cfu/ml). The strain LR32 had better tolerance to acidic pH than the control strain *L rhamnosus* GG with a difference of two more log units of growth at pH 2.5, and one more unit at pH 2 compared with this last strain.

### 3.3. Resistance to bile salts

In this case, the assay was performed only using the strain *L. reuteri* LR32. A bovine bile derivative (Ox-gall) essentially made up of conjugated bile salts was used. From a suspension of the strain LR32 in sterile saline solution, MRSC plates supplemented with increasing concentrations of Ox-gall (between 0.25 and 4%) were inoculated in duplicate. After 48 h of incubation at 37°C, the presence of growth in the plates with bile was observed. The strain of *L. reuteri* LR32 had a good resistance to bile, growing in all the concentrations of Ox-gall used.

### B. Antimicrobial capacity of the strain L. reuteri LR32

### Example 4. Anti-Helicobacter activity of the strain L. reuteri LR32 isolated from the human stomach and of other strains used as reference

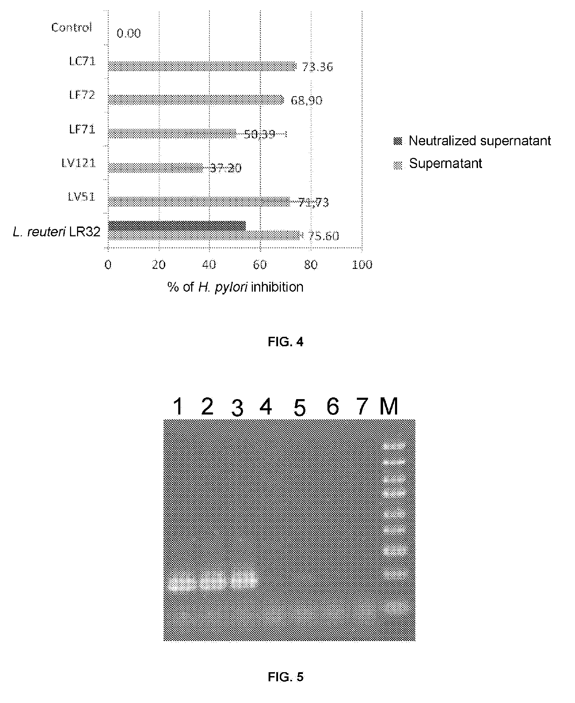

The antagonic activity of the strain LR32 and of other strains of lactobacilli isolated from the human stomach against the gastric pathogen *H. pylori* was evaluated by means of inhibition assays in liquid medium. To that end, the strain of *Helicobacter pylori* DSMZ 10242 was grown in Brain Heart Infusion (BHI) medium supplemented with 10% fetal bovine serum in microaerophylic conditions (CampyGen system, Oxoid) in 96-well plates for 3 days at 37°C, the optical density at 600 nm (OD₆₀₀) being recorded in a spectrophotometer. The increase in the OD₆₀₀ in control wells with the pathogen was compared with that of those in which 45 µl of the supernatant (neutralized at pH 6.6 and/or non-neutralized) of the strains of lactobacilli grown in Elliker medium were also added. The inhibition assays were repeated two times using independent cultures and the results were expressed as percentage of inhibition. The Elliker medium was used as negative control. The supernatants were assayed in triplicate, comparing the effect with that caused by the other gastric strains of lactobacilli, both the effect of the neutralized and non-neutralized supernatants. In this manner, it was found that the strain *L. reuteri* LR32 had a significant anti-H. *pylori* activity compared with other strains of lactobacilli of the gastric environment (see Figure 4), showing percentages of reduction in pathogen of 55% in neutralized supernatant and 75% in non-neutralized supernatant. The differences were particularly significant in the case of neutralized supernatants, in which case inhibitory effects were only observed in the strain LR32 (55% inhibition) and not in the rest of the gastric lactobacillus tested (0%).

### Example 5. Capacity of the strain L. reuteri LR32 isolated from the human stomach to produce reuterin

The presence of a key gene linked to the production of reuterin was proven by means of PCR (see Figure 5). Specifically, the gene encoding glycerol dehydratase was partially amplified and sequenced in the strain LR32. Reuterin is a potent antimicrobial agent produced during the anaerobic metabolism of glycerol, with inhibitory activity even against Gram-negative microorganisms such as *H. pylori.*

### Example 6. Production of hydrogen peroxide in the strain of L. reuteri LR32 isolated from the human stomach and of other strains used as reference

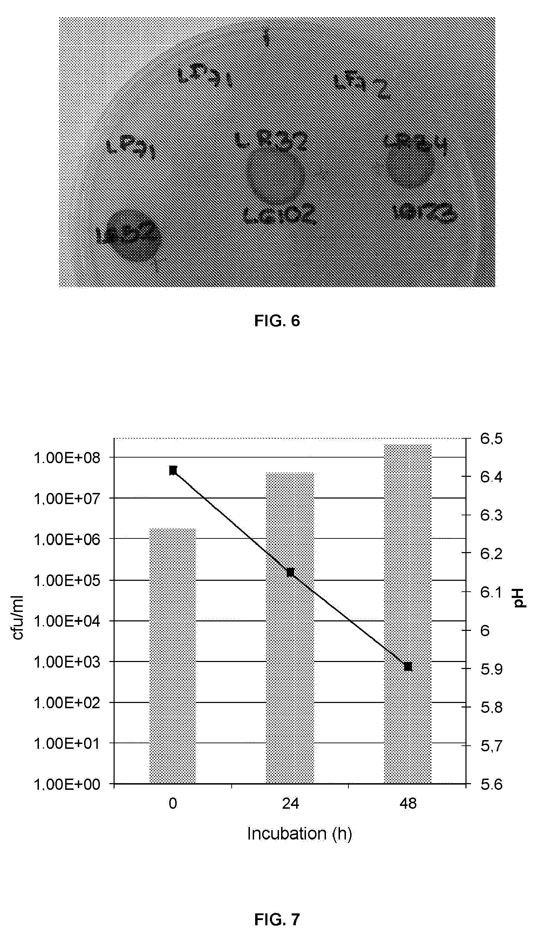

The production of hydrogen peroxide (H₂O₂) was analyzed in MRSC plates supplemented with the tetramethylbenzidine (TMB) substrate and the horseradish peroxidase (HRP) enzyme. The peroxidase oxidizes the TMB substrate in the presence of H₂O₂ forming a blue pigment in the colony producing oxygenated water. The presence of colored pigment in the strain of LR32 was clearly shown after incubation of the plates at 37°C in conditions of aerobiosis and anaerobiosis (see Figure 6). Compared with other tested strains of gastric lactobacilli of different species, only the two strains of *L. reuteri* (including LR32) and another strain of *Lactobacillus vaginalis* were capable of producing H₂O₂ in aerobic and anaerobic conditions.

### C. Anti-oxidative capacity of the strain of L. reuteri LR32

### Example 7. Anti-oxidative activity of the strain of L. reuteri LR32 isolated from the human stomach and of other strains used as reference

The anti-oxidative activity of the strain LR32 was assessed by means of the linolenic acid test consisting of observing if the microorganism somehow prevents lipid peroxidation. The test measures thiobarbituric acid reactive substances (TBARS). The tests were performed both with whole cells and with cell extracts (obtained by means of mechanical lysis in a "cell disruptor"). The reactions were essentially carried out following the procedure described by Kullisaar et al. (2002, Int. J. Food Microbiol. 72:215-224), with 45 µl of sample and measuring the absorbance at 534 nm in a spectrophotometer. The results are expressed as the percentage of inhibition of oxidation (Table 1). Both the whole cells and the cell extracts of LR32 prevented lipid peroxidation with percentages of total anti-oxidative activity (TAA) above 20%; greater than those of other strains of lactobacilli isolated from the human stomach and within the acceptable range of values for strains with proven anti-oxidative activity.

**Table 1.- Total anti-oxidative activity of different gastric strains of Lactobacillus, including L. reuteri LR32.**

| | Total anti-oxidative activity (% TAA) | |
|---|---|---|
| | Whole cells (%) | Cell extracts (%) |
| LG52 | 5 ± 2 | 3 ± 1 |
| LG102 | 16 ± 4 | 3 ± 1 |
| LG123 | 0 | 0 |
| *L. reuteri* LR32 | 22 ± 5 | 23 ± 7 |
| LV121 | 0 | 0 |
| LF71 | 0 | 0 |
| LF72 | 0 | 0 |

### Example 8. The probiotic strain of L. reuteri LR32 has the capacity to grow in and to acidify UHT milk

The capacity of the strain of *L. reuteri* LR32 to grow in UHT milk was evaluated after 24 and 48 hours. To that end, a culture of the strain which was washed in saline solution (0.9% NaCl) was started in liquid medium, the concentration of the initial inoculum being adjusted around 10⁷ cfu/ml. A tube of non-inoculated milk was used as control of the fermentation. The inoculated and non-inoculated milk were incubated at 37°C in an oven with CO₂ enriched atmosphere (5%). After the incubation, the pH of the milks was determined with a GLP 22 pH-meter of the CRISON brand and counts were performed in duplicate on MRSC plates in which ten-fold dilutions of the milk samples were seeded.

## Claims

1. A probiotic strain isolated from the human stomach, with the capacity to colonize and survive in the conditions of the human gastric ecosystem, **characterized in that** it is the strain of *Lactobacillus reuteri* LR32 with accession number CECT 8395 and **in that** it comprises:
a) a percentage of adherence to the gastric epithelium greater than 5%,
b) a survival rate at pH 2, in conditions of anaerobiosis, greater than 80%, and
c) resistance to the presence of up to 4% of bile after an incubation period of 48 h at a temperature of 37°C.

2. The probiotic strain according to claim 1, **characterized in that** the survival rate at pH 2.5 in conditions of anaerobiosis is greater than 95%.

3. The probiotic strain according to any one of claims 1 and 2, **characterized by** a total *in vitro* anti-oxidative capacity greater than 20%.

4. The probiotic strain according to any one of claims 1 to 3, **characterized by** comprising:
i) capacity to produce reuterin, and
ii) capacity to produce hydrogen peroxide in conditions of aerobiosis and anaerobiosis.

5. The probiotic strain according to any one of claims 1 to 4, **characterized by** comprising capacity to inhibit *Helicobacter pylori.*

6. The probiotic strain according to any one of claims 1 to 5, **characterized in that** it is a cell of the probiotic strain or a cell fragment of the probiotic strain present in the form of concentrate or lyophilisate.

7. Use of the probiotic strain according to any one of claims 1 to 6 for preventing and/or alleviating gastric pathologies derived from microbial dysbioses.

8. Use of the probiotic strain according to claim 7, where the gastric pathologies are caused by *H. pylori.*

9. Use of the probiotic strain according to any one of claims 1 to 6 for preparing a food composition, a food supplement or a biotherapeutic composition useful for preventing and/or alleviating gastric pathologies derived from microbial dysbioses.

10. Use of the probiotic strain according to claim 9, where the gastric pathologies are caused by *H. pylori.*

11. A probiotic food composition, food supplement or biotherapeutic composition which comprises the probiotic strain according to any one of claims 1 to 6 and is useful for preventing and/or alleviating gastric pathologies derived from microbial dysbioses.

12. The probiotic food composition, food supplement or biotherapeutic composition according to claim 11, where the gastric pathologies are caused by *H. pylori.*

13. A food composition according to any one of claims 11 and 12, **characterized in that** it is a dairy product fermented by the probiotic strain according to any one of claims 1 to 6.

14. The food composition according to claim 13, **characterized in that** it additionally uses other microorganisms for fermentation.

15. The food supplement or biotherapeutic composition according to any one of claims 11 and 12, **characterized in that** they comprise any one of the following presentations, capsule, lyophilisate, liquid, pill or gel.


SEQUENCE LISTING

<110> CONSEJO SUPERIOR DE INVESTIGACIONES CIENTÍFICAS

<120> PROBIOTIC STRAIN OF LACTOBACILLUS REUTERI EXHIBITING ANTI-HELICOBACTER
ACTIVITY, PROBIOTIC OR BIOTHERAPEUTIC PRODUCT COMPRISING SAID STRAIN AND THE
USE THEREOF

<140> PCT/ES2014/070666
<141> 2014-08-21

<150> ES P201331271
<151> 2013-08-23

<160> 1

<170> BiSSAP 1.2

<210> 1
<211> 450
<212> DNA
<213> Lactobacillus reuteri LR32

<220>
<221> source
<222> 1..450
<223> /organism="Lactobacillus reuteri LR32"
      /note="Partial sequence of the gene encoding 16S rRNA of
      L. reuteri strain LR32"
      /mol_type="unassigned DNA"

<400> 1
gtcgtacgca ctggcccaac tgattgatgg tgcttgcacc tgattgacga tggatcacca      60

gtgagtggcg gacgggtgag taacacgtag gtaacctgcc ccggagcggg ggataacatt     120

tggaaacaga tgctaatacc gcataacaac aaaagccgca tggcttttgt ttgaaagatg     180

gctttggcta tcactctggg atggacctgc ggtgcattag ctagttggta aggtaacggc     240

ttaccaaggc gatgatgcat agccgagttg agagactgat cggccacaat ggaactgaga     300

cacggtccat actcctacgg gaggcagcag tagggaatct tccacaatgg gcgcaagcct     360

gatggagcaa caccgcgtga gtgaagaagg gtttcggctc gtaaagctct gttgttggag     420

aagaacgtgc gtgagagtaa ctgttcacgc                                      450